# EUROPÄISCHE PATENTSCHRIFT

(11) **EP 2 468 317 B1**
(45) Veröffentlichungstag und Bekanntmachung des Hinweises auf die Patenterteilung: **07.09.2016**
(21) Anmeldenummer: 11009385.3
(22) Anmeldetag: 26.11.2011
(51) Int. Cl.: B05D 1/18, A61L 27/54

(54) **Beschichtungsvorrichtung**
Coating device
Dispositif de revêtement

(30) Priorität: 23.12.2010 DE 102010055560
(43) Veröffentlichungstag der Anmeldung: 27.06.2012
(73) Patentinhaber: Heraeus Medical GmbH, 61273 Wehrheim (DE)
(72) Erfinder: Vogt, Sebastian, 99092 Erfurt (DE); Kühn, Klaus-Dieter, 35041 Marburg-Elnhausen (DE)
(74) Vertreter: Heraeus IP

(56) Entgegenhaltungen:
- EP-A1- 0 623 349
- DE-A1- 10 351 150
- US-A1- 2003 078 242
- US-A1- 2003 229 401
- US-A1- 2005 170 070
- US-A1- 2006 029 722
- US-A1- 2006 251 824
- US-B1- 6 451 373

## Beschreibung

Die Erfindung betrifft eine Vorrichtung zur zumindest bereichsweisen Beschichtung eines medizinischen Implantats mit einem solchen Verfahren.

Die Beschichtung von medizinischen Implantaten mit pharmazeutischen Wirkstoffen hat in den letzten Jahren zunehmend Beachtung gefunden. Eine zentrale Anwendung von Beschichtungsverfahren liegt dabei im antibiotischen Schutz der Oberfläche von Implantatmaterialien. Eine weitere wichtige Anwendung ist in der Verbesserung der Oberflächenkompatibilität von nicht zu zementierenden medizinischen Implantaten zum besseren Anwachsen von Knochenmaterial zu sehen.

Die Implantation von Gelenkendoprothesen und auch von Osteosynthesematerialien ist immer mit einer gewissen Gefahr einer mikrobiellen Kontamination verbunden. Wenn es mikrobiellen Keimen gelingt, sich an der Implantatoberfläche anzusiedeln, kann es zur Ausbildung einer post-operativen Osteitis/Osteomyelitis kommen. Die Osteitis/Osteomyelitis stellt eine schwerwiegende Komplikation für den Patienten dar, die zusätzlich mit erheblichen Kosten verbunden ist.

Seit Jahrzehenten wird mit klinischem Erfolg bei zementierten Gelenkendoprothesen mit Gentamicin dotierter PMMA-Knochenzement verwendet. Das im Knochenzement enthaltende Breitbandantibiotikum Gentamicin schützt die Oberfläche des Knochenzementes wirksam gegen bakterielle Infektionen.

Bei nicht-zementierten Gelenkendoprothesen und bei Osteosynthesematerialien wurde eine Reihe von Lösungswegen vorgeschlagen, um ebenfalls einen lokalen antibiotischen Schutz der Implantatoberflächen zu erreichen.

So wurde in mehreren Patentdokumenten die Verwendung von in Wasser gering löslichen Antibiotika-Salzen beschrieben. Exemplarisch seien dafür die EP 0 623 349 A1, EP 1 470 829 A1, EP 1 374 923 A2, DE 101 42 465 A1 und DE 44 04 018 A1 genannt. Diese in Wasser gering löslichen Salze lösen sich unter Freisetzung der enthaltenen Antibiotika durch Einwirkung von Körperflüssigkeiten auf. Vorteilhaft ist die protrahierte Wirkstofffreisetzung. Nachteilig ist jedoch die aufwendige Herstellung dieser Salze.

Alternativ dazu ist es auch möglich, in Wasser lösliche Antibiotika-Salze zu verwenden. Das Problem liegt dabei darin, das Antibiotikum auf der Implantatoberfläche zu fixieren.

Aus US 6451373 B1 ist ein Verfahren zur Bildung einer therapeutischen Beschichtung bekannt. Das Implantat wird in eine Lösung eingetaucht und die überschüssige Flüssigkeit wird mit einem porösen, fusselfreien Tuch abgewischt.

Die Mehrzahl der bisherigen beschriebenen Beschichtungen ist vorzugsweise für die Fertigung von beschichteten Implantaten unter industriellen Bedingungen bestimmt. Das bedeutet, dass die industrielle Beschichtung dieser Implantate nur mit wenigen für die Großanwendung relevanten Wirkstoffen erfolgen kann, um eine wirtschaftliche industrielle Fertigung mit entsprechenden hohen Stückzahlen gewährleisten zu können.

Insbesondere bei antibiotischen Beschichtungen ist es jedoch im Hinblick auf die sich zunehmend problematisch gestaltende Resistenzsituation und dem dabei vermehrten Auftreten von multiresistenten Keimen, wie MRSA und MRSE von Interesse, genau auf die jeweiligen Keime abgestimmte Antibiotika oder Antibiotika-Kombinationen zur Beschichtung von Revisionsprothesen im Zuge des einzeitigen oder zweizeitigen septischen Gelenkprothesenwechsels einzusetzen, um einen initialen antibiotischen Schutz der Implantatoberflächen zu gewährleisten. Nachteilig ist hieran, dass die Verfahren zur Beschichtung der medizinischen Implantate relativ aufwendig sind. Eine variable kurzfristige Anwendung ist nicht möglich. Es müssen für verschiedene Situationen verschiedene beschichtete medizinische Implantate bereitgehalten werden, um den Bedürfnissen der unterschiedlichen Patienten gerecht zu werden. Dies erfordert eine umfangreiche Lagerhaltung und verhindert ungewöhnliche Mischungen für spezielle Fälle. Die Aufgabe der Erfindung besteht also darin, die Nachteile des Stands der Technik zu überwinden. Insbesondere soll eine Vorrichtung hierfür bereitgestellt werden, mit denen eine medizinische Prothese beschichtet werden kann, ohne dass dadurch der Ablauf einer Operation (OP) gestört wird. Es sollen möglichst viele verschiedene medizinische Implantate mit der Vorrichtung beschichtet werden können. Auch soll die Vorrichtung variabel einsetzbar sein, so dass sie an die medizinischen Notwendigkeiten, insbesondere an eine für den Patienten geeignete Medikamentierung anpassbar sind. Der in Operationssälen gebotenen Reinheit soll ebenfalls Rechnung getragen werden.

Die Aufgabe der Erfindung besteht ferner darin, eine einfache Beschichtungsvorrichtung zu entwickeln, die es dem OP-Personal erlaubt, unter OP-Bedingungen Implantate mit geringstem Aufwand zu beschichten. Die Vorrichtung soll weiterhin so gestaltet sein, dass möglichst kein überschüssiges Material zur Herstellung der Beschichtung den OP-Bereich kontaminieren kann. Eine weitere Aufgabe besteht darin, dass die Vorrichtung besonders zur Beschichtung von nicht-zementierten Gelenkendoprothesen und Osteosynthesematerialien geeignet sein soll.

Es wird auch beschrieben dass ein medizinisches Implantat mit einer zu beschichtenden Oberfläche bereitgestellt wird, die zu beschichtende Oberfläche des medizinischen Implantats in eine Flüssigkeit umfassend zumindest eine pharmazeutisch aktive Substanz getaucht wird, wobei durch das Eintauchen Flüssigkeit auf die Oberfläche des medizinischen Implantats übertragen wird, und die zu beschichtende Oberfläche des medizinischen Implantats aus der Flüssigkeit herausgezogen wird, wobei ein Teil der Flüssigkeit auf der zu beschichtenden Oberfläche des medizinischen Implantats haften bleibt.

Solche Verfahren erfolgen vor dem Einsetzen der medizinischen Implantate. Die Verfahren finden also "ex vivo" statt.

Unter einer pharmazeutisch aktiven Substanz sind erfindungsgemäß pharmazeutisch wirksame Mittel und Mittel zu verstehen, die pharmakologisch wirken, sowie solche, die eine pharmakologische Wirkung unterstützen oder sonst in irgendeiner Weise die Selbstheilungskräfte des Körpers unterstützen. Beispiele sind hierfür Antibiotika, organische Antiseptika, Kupfersalze, Kupferoxid, Galliumsalze, Strontiumsalze, Lithiumsalze, Silbersalze, Silberoxid, Bisphosphonate, Wachstumsfaktoren, Steroidhormone, nichtsteroidale Hormone, Hämostyptika, Antiphlogistika, Plasmide, Cosmide, lineare DNA und deren Mischungen.

Unter einem Eintauchen ist nicht nur ein Eintauchen in einer großen Menge der Flüssigkeit zu verstehen. Wenn die Form des Flüssigkeitsbehältnisses an die Form des zu beschichtenden medizinischen Implantats angepasst ist, kann auch eine geringe Menge der Flüssigkeit ausreichen, um die gesamte zu beschichtende Oberfläche des medizinischen Implantats zu beschichten. Die Flüssigkeit drückt sich dann zwischen dem medizinischen Implantat und dem Flüssigkeitsbehältnis nach oben.

Es wird auch beschrieben dass das zu beschichtende Implantat gegebenenfalls mehrfach in die Flüssigkeit eingeführt und herausgezogen wird.

Ferner kann vorgesehen sein, dass das zu beschichtende medizinische Implantat aus Hüftgelenkendoprothesen, Schultergelenkendoprothesen, Ellbogenendoprothesen, Marknägeln und Osteosyntheseplatten ausgewählt wird.

Es kann auch vorgesehen sein, dass die Flüssigkeit eine wässrige Lösung eines Antibiotikums umfasst, bevorzugt eine wässrige Gentamicinsulfat-Lösung mit einem Gentamicinsulfat-Gehalt von 10,0 bis 88,0 Gewichtsprozent verwendet wird, wobei ganz besonders eine Gentamicinsulfat-Lösung mit einem Gentamicinsulfat-Gehalt von 75,0 bis 80,0 Gewichtsprozent bevorzugt verwendet wird. Diese Gentamicinsulfat-Lösung hat eine ölig-viskose Konsistenz und haftet sehr gut auf Metalloberflächen.

Es kann dabei ferner vorgesehen sein, dass pharmazeutisch übliche Stabilisatoren in den Gentamicinsulfat-Lösungen enthalten sind. Diese verbessern die Haltbarkeit und damit die Verwendbarkeit der aufzutragenden Flüssigkeit.

Es kann auch die Verwendung von anderen Aminoglykosid-Antibiotika-Lösungen, wie wässrige Lösungen des Tobramycinsulfats, des Amikacinsulfats, des Netilmicinsulfats und des Sisomycinsulfats als Flüssigkeit oder Flüssigkeitsbestandteile vorgesehen sein. Es ist auch möglich, wässrige Lösungen des Vancomycins, des Dalbavancins, des Ramoplanins, des Daptomycins, des Moxifloxacins, des Clindamycins und des Lincomycins einzusetzen.

Ferner kann die Verwendung von Kombinationen von Lösungen verschiedener Antibiotika als Flüssigkeit vorgesehen sein. Beispielhaft sind hierbei die Zweifachkombinationen von Gentamicinsulfat mit Vancomycinhydrochlorid, die Zweifachkombination von Daptomycin mit Gentamicinsulfat und die Zweifachkombination von Gentamicinsulfat mit Clindamycin sowie die Dreifachkombination Gentamicinsulfat mit Vancomycinhydrochlorid und Clindamycinhydrochlorid.

Des Weiteren kann vorgesehen sein, dass als Flüssigkeit Antiseptika-Lösungen verwendet werden, insbesondere Lösungen des Chlorhexidindigluconats, des Octenidindihydrochlorids und des Polyhexanids.

Gemäß einer besonders vorteilhaften Ausgestaltung kann vorgesehen sein, dass die Flüssigkeit in einem Behälter mit einer Öffnung bereitgestellt wird, wobei das medizinische Implantat zur Beschichtung der zu beschichtenden Oberfläche durch die Öffnung eingeführt wird.

Solche Verfahren können sich auch dadurch auszeichnen, dass das medizinische Implantat durch eine Membran gestoßen wird oder eine Membran geöffnet wird, bevor das medizinische Implantat in die Flüssigkeit getaucht wird, wobei die Membran die Flüssigkeit zumindest bereichsweise abdeckt, vorzugsweise die Membran die Flüssigkeit im Behälter vollständig abdeckt. Durch diese beiden Maßnahmen kann das Verfahren leicht an unterschiedlichen Orten eingesetzt werden, da die zu verwendende Vorrichtung leicht transportabel ist.

Eine weitere Ausgestaltung des Verfahrens kann vorsehen, dass eine zur Behandlungssituation passende Flüssigkeit bereitgestellt wird.

Auch kann vorgesehen sein, dass ein zur Behandlungssituation passendes Antibiotikum oder Antibiotika-Gemisch in die Flüssigkeit eingebracht wird. Durch diese beiden Maßnahmen kann auf die konkrete Behandlungssituation beim jeweiligen Patienten eingegangen werden.

Dabei kann vorgesehen sein, dass das medizinische Implantat vor dem Eintauchen in die Flüssigkeit in einen Behälter eingeführt wird, in dem sich die Flüssigkeit befindet und nach der Übertragung der Flüssigkeit auf das medizinische Implantat aus dem Behälter herausgezogen wird.

Auch kann vorgesehen sein, dass das medizinische Implantat durch eine Membran gestoßen wird oder eine Membran geöffnet wird, bevor das medizinische Implantat in die Flüssigkeit getaucht wird, wobei die Membran die Flüssigkeit zumindest bereichsweise abdeckt, vorzugsweise die Membran die Flüssigkeit im Behälter abdichtet. Die Membran verhindert eine Kontamination der Flüssigkeit vor der Anwendung. Durch das Durchstoßen der Membran wird sichergestellt, dass die schützende Membran erst kurz vor der Anwendung geöffnet wird. Die Membran sollte dazu derart aufgebaut sein, dass keine Fetzen oder andere Teile der Membran in die Flüssigkeit gelangen können oder am medizinischen Implantat haften.

Besonders bevorzugt ist, dass ein Teil der übertragenen Flüssigkeit abgestreift wird, insbesondere beim Herausziehen des medizinischen Implantats aus dem Behälter, vorzugsweise an einem dafür vorgesehenen Abstreifer. Hierdurch kann eine Kontamination der Umgebung, also insbesondere eines OP-Bereichs, mit der Flüssigkeit verhindert oder zumindest reduziert werden. Dies ist vor allem bei der Verwendung von Antibiotika geboten, da so der Entwicklung resistenter Keime im OP-Bereich vorgebeugt werden kann.

Ferner kann vorgesehen sein, dass zumindest 50% der Oberfläche des medizinischen Implantats, vorzugsweise zumindest 80%, besonders bevorzugt zumindest 90% der Oberfläche des medizinischen Implantats beschichtet werden.

Manche Verfahren können sich auch dadurch auszeichnen, dass eine zweite Flüssigkeit, vorzugsweise umfassend zumindest eine pharmazeutisch aktive Substanz, über ein Übertragungsmittel auf die Oberfläche des medizinischen Implantats übertragen wird, bevor das medizinische Implantat eingetaucht wird.

Dabei kann vorgesehen sein, dass das medizinische Implantat über ein elastisch deformierbares Übertragungsmittel gestrichen wird, wobei beim Überstreichen des Übertragungsmittels die zweite Flüssigkeit vom Übertragungsmittel auf die zu beschichtenden Oberfläche des medizinischen Implantats übertragen wird. Durch die Verwendung eines elastisch deformierbaren Übertragungsmittels wird erreicht, dass die durch das Übertragungsmittel aufgetragene zweite Flüssigkeit auch auf einem unregelmäßig geformten medizinischen Implantat großflächig aufgetragen werden kann. Besonders bevorzugt ist das Übertragungsmittel auch porös, wobei die zweite Flüssigkeit in den Poren des Übertragungsmittels gespeichert wird. Das Übertragungsmittel kann dann Oberhalb der (ersten) Flüssigkeit angeordnet werden, ohne dass die zweite Flüssigkeit in die (erste) Flüssigkeit tropft. Besonders in Kombination mit einer Membran zum Abdecken der (ersten) Flüssigkeit ist dies vorteilhaft.

Es kann auch vorgesehen sein, dass nach dem Übertragen der Flüssigkeit auf das medizinische Implantat ein Pulver auf die benetzte Oberfläche des medizinischen Implantats aufgebracht wird, bevorzugt das medizinische Implantat in ein Pulver eingetaucht wird, wobei das Pulver vorzugsweise zumindest eine knochenwachstumsfördernde Substanz umfasst.

Um den beschichteten Bereich und die Vollständigkeit der Beschichtung sichtbar zu machen, kann vorgesehen sein, dass die Flüssigkeit eingefärbt wird, so dass der beschichtete Bereich des medizinischen Implantats farblich kenntlich gemacht wird.

Dabei kann vorgesehen sein, dass die Vollständigkeit der Beschichtung des zu beschichtenden Bereichs anhand der Färbung geprüft wird.

Es kann ferner vorgesehen sein, dass das Verfahren so oft wiederholt wird, bis eine vollständige Beschichtung der zu beschichtenden Oberfläche des medizinischen Implantats erreicht wird. Insbesondere im Zusammenhang mit einer Färbung der Flüssigkeit und der Prüfung der Vollständigkeit der Beschichtung mit Hilfe der Färbung ist dies vorteilhaft, um eine ausreichend beschichtetes medizinisches Implantat zu erzeugen.

Es kann vorteilhaft sein, wenn der Behälter mit dem eingeschobenen Implantat geschlossen und/oder abgedichtet wird. Dies kann durch Schließen eines dafür vorgesehenen Deckels geschehen.

Anschließend kann vorgesehen sein, dass der Behälter mit dem darin eingeschlossenen Implantat kurz geschüttelt wird.

Die Aufgabe der Erfindung wird auch gelöst durch eine Vorrichtung zur zumindest bereichsweisen Beschichtung eines medizinischen Implantats mit einem solchen Verfahren, wobei die Vorrichtung einen Behälter umfasst, der eine Flüssigkeit aufweist, die zumindest eine pharmazeutisch aktive Substanz enthält, und der Behälter eine Öffnung zum Einführen und Herausnehmen des medizinischen Implantats umfasst.

Dabei kann vorgesehen sein, dass die Öffnung durch einen abziehbaren Deckel verschlossen ist. Hierdurch kann eine Verunreinigung des Inneren des Behälters vermieden werden.

Eine besonders vorteilhafte Ausgestaltung der Erfindung kann vorsehen, dass die Vorrichtung einen Abstreifer umfasst, der vorzugsweise im Bereich der Öffnung, insbesondere zwischen der Öffnung und der Flüssigkeit, angeordnet ist.

Dabei kann vorgesehen sein, dass der Abstreifer scheibenförmig ist und mindestens einen Einschnitt umfasst, der die Oberseite und die Unterseite der Scheibe verbindet. Durch diesen mindesten einen Einschnitt kann das Implantat in die Vorrichtung eingebracht werden. Besonders vorteilhaft ist es, wenn radiale Einschnitte in dem Abstreifer ausgebildet sind. Dies macht es möglich, den gesamten äußeren Umfang der Implantate nach Beendigung der Beschichtung abzustreifen und damit überschüssige Mengen der Lösung oder Suspension von der beschichteten Implantatoberfläche zu entfernen. Weiterhin ist es dadurch möglich, die Freisetzung von Tröpfchen der Flüssigkeit, die beim Herausziehen des Implantats aus der Flüssigkeit entstehen können, wirksam zurückzuhalten. Damit wird die Kontamination des OPs weitgehend vermieden.

Es kann des Weiteren vorgesehen sein, dass der Abstreifer als Kegelmantel oder als Halbkugelfläche ausgebildet ist, wobei die Kegelspitze oder die Halbkugel in Richtung der Flüssigkeit ausgerichtet ist und vorzugsweise der Kegelmantel oder die Halbkugel mindestens einen Einschnitt enthalten, der die Oberseite und die Unterseite des Abstreifers verbindet.

Erfindungsgemäß kann auch vorgesehen sein, dass über der Flüssigkeit ein Übertragungsmittel angeordnet ist, mit dem eine zweite Flüssigkeit auf das medizinische Implantat übertragbar ist, wobei die zweite Flüssigkeit in dem Übertragungsmittel enthalten ist.

Dabei kann vorgesehen sein, dass das Übertragungsmittel Poren umfasst und in den Poren des Übertragungsmittels die zweite Flüssigkeit, vorzugsweise in Form einer Lösung und/oder Suspension enthalten ist, wobei in der zweiten Flüssigkeit vorzugsweise eine zweite pharmazeutisch aktive Substanz enthalten ist.

Eine Weiterbildung der Erfindung sieht vor, dass das Übertragungsmittel zumindest eine Walze, zumindest eine drehbare Kugel und/oder zumindest einen Schwamm umfasst, mit der oder dem oder mit denen die zweite Flüssigkeit auf die zu beschichtende Oberfläche des medizinischen Implantats übertragbar ist. Dadurch kann die Menge der zu verwendenden zweiten Flüssigkeit reduziert werden und ein ungewolltes Vermischen größerer Mengen der zweiten Flüssigkeit mit der (ersten) Flüssigkeit vermieden werden.

Gemäß einer besonders bevorzugten Ausgestaltung der Erfindung kann vorgesehen sein, dass die pharmazeutisch aktive Substanz und vorzugsweise auch die zweite pharmazeutisch aktive Substanz Antibiotika und/oder organische Antiseptika beinhaltet, so dass die zu erzeugende Beschichtung eine pharmazeutisch wirksame Dosis beinhaltet.

Ferner kann vorgesehen sein, dass die Vorrichtung einen Vakuumanschluss umfasst, der mit einer Vakuumquelle verbindbar ist und der vorzugsweise zwischen dem Abstreifer und der Flüssigkeit angeordnet ist. Dadurch kann zusätzlich durch Absaugen von Tröpfchen der Flüssigkeit und gegebenenfalls auch Pulverresten sichergestellt werden, dass keine Kontamination des OPs mit pharmazeutischen Wirkstoffen erfolgt.

Auch kann vorgesehen sein, dass der Abstreifer aus einem biokompatiblen Elastomer, thermoplastischen Kunststoff oder auch durch eine Metallfolie oder auch aus Kompositen, die aus Metall-Elastomer-Kombinationen oder Metall-Kunststoffkombinationen gefertigt sind, besteht.

Ferner kann vorgesehen sein, dass der Abstreifer als Ring ausgebildet ist, der radial zum Mittelpunkt des Behälters angeordnete Borsten enthält. Diese Borsten können aus Kunststoff gebildet sein, wobei die Borsten mechanisch so stabil und so stabil verankert sind, dass ein Abbrechen oder Ablösen der Borsten unterbleibt.

Gemäß einer Weiterentwicklung der Erfindung kann auch vorgesehen sein, dass der Abstreifer in Form von drehbaren oder nichtdrehbaren Walzen und/oder auch Kugeln ausgebildet ist, die durch elastische Verbindungsmittel mit dem Behälter verbunden sind. Durch diesen Aufbau wird eine besonders einfache Abstreifbarkeit überschüssiger Flüssigkeit und gegebenenfalls überschüssigen Pulvers erreicht.

Die Vorrichtung kann erfindungsgemäß mit einem Pulver, mit einer Wirkstofflösung und/oder Wirkstoffsuspension vorbefüllt sein, so dass das OP-Personal nur die Vorrichtung öffnen muss und anschließend sofort die Beschichtung des Implantats vornehmen kann. Vorteilhaft ist hierbei, dass der Zeitaufwand für diese Beschichtung nur im Bereich von wenigen Sekunden liegt und wertvolle OP-Zeit gespart werden kann.

Alternativ ist es möglich, eine nicht befüllte Vorrichtung direkt im OP durch Einspritzen einer Wirkstofflösung oder Wirkstoffsuspension Einfüllen und/oder eines Pulvers mit einem oder mehreren pharmazeutischen Wirkstoffen auszurüsten. Dadurch ist es im Fall der antibiotischen Beschichtung möglich, entsprechend der vorliegenden Resistenzsituation eine geeignete Auswahl eines Antibiotikums oder einer Antibiotika-Kombination vorzunehmen und damit eine Antibiogramm-gerechte Beschichtung herzustellen.

Es ist auch möglich, vor der OP nicht befüllte Vorrichtungen in der jeweiligen Klinikapotheke mit geeigneten Wirkstofflösungen oder Wirkstoffsuspensionen zu befüllen, so dass während der OP die Beschichtung ohne Zeitverzug vorgenommen werden kann.

Beispiele für verwendbare pharmazeutisch aktive Substanzen sind Antibiotika, organische Antiseptika, Kupfersalze, Kupferoxid, Galliumsalze, Strontiumsalze, Lithiumsalze, Silbersalze, Silberoxid, Bisphosphonate, Wachstumsfaktoren, Steroidhormone, nichtsteroidale Hormone, Hämostyptika, Antiphlogistika, Plasmide, Cosmide, lineare DNA und deren Mischungen.

Als Flüssigkeiten können erfindungsgemäß wässrige Lösung eines Antibiotikums, bevorzugt eine wässrige Gentamicinsulfat-Lösung mit einem Gentamicinsulfat-Gehalt von 10,0 bis 88,0 Gewichtsprozent vorgesehen sein, wobei ganz besonders eine Gentamicinsulfat-Lösung mit einem Gentamicinsulfat-Gehalt von 75,0 bis 80,0 Gewichtsprozent bevorzugt ist. Diese Gentamicinsulfat-Lösung hat eine ölig-viskose Konsistenz und haftet sehr gut auf Metalloberflächen. Pharmazeutisch übliche Stabilisatoren können weiterhin in den Gentamicinsulfat-Lösungen enthalten sein.

Im Rahmen der Efindung ist ebenfalls die Verwendung von anderen Aminoglykosid-Antibiotika-Lösungen, wie wässrige Lösungen des Tobramycinsulfats, des Amikacinsulfats, des Netilmicinsulfats und des Sisomycinsulfats. Es ist auch möglich, wässrige Lösungen des Vancomycins, des Dalbavancins, des Ramoplanins, des Daptomycins, des Moxifloxacins, des Clindamycins und/oder des Lincomycins einzusetzen. Ebenfalls im Rahmen der Erfindung ist die Verwendung von Kombinationen von Lösungen verschiedener Antibiotika. Beispielhaft sind hierbei die Zweifachkombinationen von Gentamicinsulfat mit Vancomycinhydrochlorid, die Zweifachkombination von Daptomycin mit Gentamicinsulfat und die Zweifachkombination von Gentamicinsulfat mit Clindamycin sowie die Dreifachkombination Gentamicinsulfat mit Vancomycinhydrochlorid und Clindamycinhydrochlorid. Weiterhin ist es möglich, anstelle von Antibiotika-Lösungen auch Antiseptika-Lösungen zu verwenden. Beispielhaft sind hierbei Lösungen des Chlorhexidindigluconats, des Octenidindihydrochlorids und des Polyhexanids.

Ebenfalls im Rahmen der Erfindung ist die Verwendung von Lösungen von Antibiotika und Antiseptika, die als Lösungsmittel organische Lösungsmittel oder Kombinationen von organischen Lösungsmitteln oder auch Kombinationen von organischen Lösungsmitteln und Wasser enthalten.

Es ist dadurch möglich, zum Beispiel auch in Wasser gering lösliche Antibiotika-Salze, wie Laurate, Myristate, Palmitate und Stearate zu verwenden. Daneben können auch in Wasser gering lösliche Antibiotika oder Antibiotika-Salze in Form von wässrigen Suspensionen eingesetzt werden.

Vorzugsweise enthält das gegebenenfalls verwendete Pulver eine knochenwachstumsfördernde Substanz. Diese knochenwachstumsfördernde Substanz kann beispielsweise aus der Gruppe ausgewählt sein, die aus ß-Tricalciumphosphat, α-Tricalciumphosphat, amorphem Calciumphosphat, Tetracalciumphosphat, Octacalciumphosphat, Hydroxylapatit, Fluorapatit, Calciumsulfat-Hemihydrat, Calciumsulfat-Dihydrat, wasserfreiem Calciumsulfat, pulverförmigen Antibiotika, organischen Antiseptika, Kupfersalzen, Kupferoxid, Galliumsalzen, Strontiumsalzen, Lithiumsalzen, Silbersalzen, Silberoxid, Bisphosphonaten, Wachstumsfaktoren, Steroidhormonen, nichtsteroidalen Hormonen, Hämostyptika, Antiphlogistika, Plasmiden, Cosmiden, linearer DNA sowie Mischungen davon besteht. In dem Pulver können auch Komplexbildner oder auch Salze enthalten sein, die mit den vom Abstreifer auf die Implantatoberfläche übertragenen pharmazeutischen Wirkstoffen in Wasser gering lösliche komplexe oder Salze bilden. So kann zum Beispiel Teicoplanin im Pulver enthalten sein, das mit Gentamicin oder anderen kationischen Antibiotika in Wasser gering lösliche Komplexe bildet. Es ist auch beispielsweise möglich, dass in dem Pulver N-Methyl-Glucamoniumsalze von Fettsäuren oder von Alkylsulfaten enthalten sind, die bei Kontakt mit wässrigen Lösungen von kationischen Antibiotika infolge eines reziproken Salzaustauschs in Wasser gering lösliche Fettsäuresalze oder Alkylsulfate der Antibiotika bilden können. Auf diese Weise ist es möglich, in Wasser gering lösliche Komplexe oder Salze von pharmazeutischen Wirkstoffen, insbesondere von Antibiotika, auf die Implantatoberfläche aufzubringen.

Besonders vorteilhaft ist es, wenn reaktive anorganische Pulver, wie amorphisiertes Calciumphosphat, Tetracalciumphosphat und Calciumsulfat-Hemihydrat verwendet werden, die in Gegenwart von Wasser aushärten. Dadurch ist es möglich, stabile Beschichtungen auszubilden. Die Aushärtung innerhalb von wenigen Sekunden kann dabei erreicht werden, indem zum Beispiel bei Verwendung von Calciumsulfat-Hemihydrat als Pulver geringe Mengen von Calciumsulfat-Dihydrat als Keimbildner sowie Ammoniumsulfat, Natriumsulfat oder Kaliumsulfat als Beschleuniger zu dem Calciumsulfat-Hemihydrat zugegeben werden. Vorteilhaft ist weiterhin die Verwendung von ß-Tricalciumphosphat, α-Tricalciumphosphat und Tetracalciumphosphat, die durch Einwirkung von wässrigen Säuren, insbesondere von wässrigen Lösungen der Äpfelsäure, der Weinsäure und der Citronensäure, innerhalb von wenigen Sekunden aushärten.

Erfindungsgemäß ist es ferner, dass die Vorrichtung als Arzneimittel oder als Medizinprodukt bereitgestellt wird.

Auch eine Kombination der erfindungsgemäßen Vorrichtung mit einem medizinischen Implantat könnte angeboten werden. Diese Kombination wird aus der Vorrichtung und dem Implantat gebildet, wobei diese Kombination eine Mindestlebensdauer von 0,1 Sekunden hat. Die Kombination entsteht besteht während des Beschichtungsprozesses.

Der Erfindung liegt die überraschende Erkenntnis zugrunde, dass eine Flüssigkeit, mit der ein medizinisches Implantat beschichtet werden soll, auch kurz vor seiner Anwendung einfach durch Eintauchen des Implantats in die Flüssigkeit auf dem medizinischen Implantat aufgetragen werden kann. Das einfach gestaltete Verfahren und die Vorrichtung hierfür stellen dabei die Anwendbarkeit auch im OP-Bereich sicher.

Für einen initialen antibiotischen Schutz reicht es aus, wenn für einen Zeitraum von 24 bis 72 Stunden hinreichend hohe Antibiotikum- oder Antibiotika-Konzentrationen an den Implantatoberflächen vorhanden sind. Daher kann auch bei der lokalen Einbringung von einfachen in Wasser löslichen Antibiotika in eine Flüssigkeit ein ausreichender temporärer, lokaler, antibiotischer Schutz des medizinischen Implantats erzielt werden.

Anstatt also das medizinische Implantat lange im Voraus bei der Herstellung zu beschichten, kann es auch unmittelbar vor dem Einsetzen beschichtet werden. Dadurch können auch relativ kurzlebige Beschichtungen verwendet werden. Zudem kann sogar eine noch flüssige Schicht verwendet werden, was neue Anwendungsgebiete eröffnet und neue Wirkstoffe zugänglich macht.

Im Folgenden werden Ausführungsbeispiele der Erfindung anhand von zwei schematisch dargestellten Figuren erläutert, ohne jedoch dabei die Erfindung zu beschränken. Dabei zeigt:
Figur 1: eine schematische Querschnittansicht einer erfindungsgemäßen Vorrichtung und
Figur 2: eine schematische perspektivische Ansicht einer erfindungsgemäßen Vorrichtung.

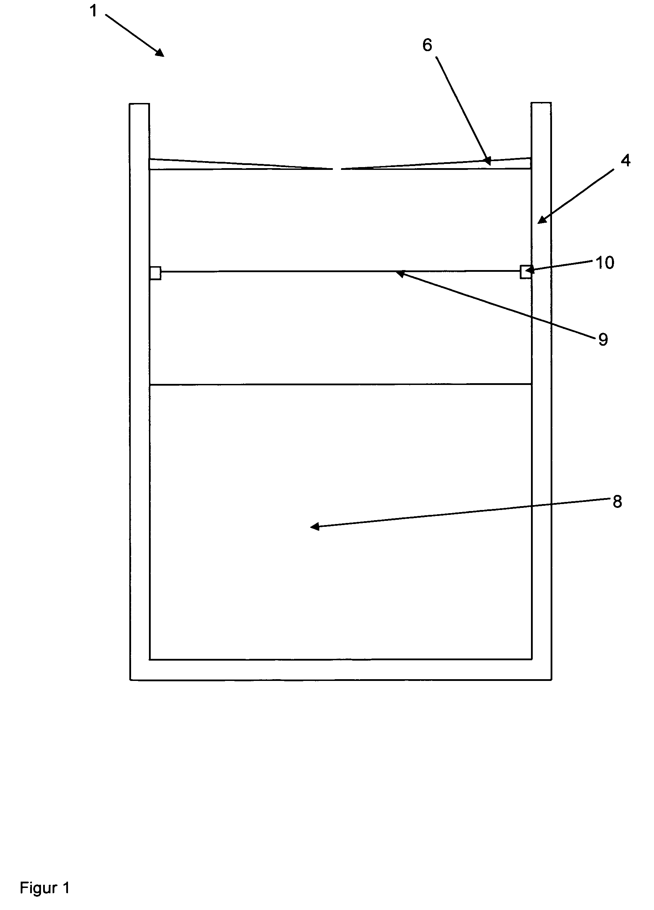

Figur 1 zeigt eine schematische Querschnittansicht einer erfindungsgemäßen Vorrichtung 1. Die Vorrichtung 1 umfasst einen Behälter 4 in Form eines oben offenen Topfs. Die Seitenwände des Behälters 4 sind zylindrisch und von gleichmäßiger Dicke. Im Bereich der Öffnung, kurz unterhalb der Öffnung ist im Inneren des Behälters 4 ein Abstreifer 6 angeordnet, der die Öffnung abschließt.

Der Boden und die Seitenwände des Behälters 4 und der Abstreifer 6 sind aus einem hydrophoben Material gefertigt oder mit einer hydrophoben Schicht beschichtet. Ausgehend von der Mitte des Abstreifers 6 ist der Abstreifer 6 in acht Richtungen geschlitzt beziehungsweise eingeschnitten.

Die acht Schlitze / Einschnitte (nicht gezeigt) reichen nicht bis zu den Seitenwänden des Behälters 4 und sollen das Einführen eines medizinischen Implantats durch den Abstreifer 6 ermöglichen. Der Abstreifer 6 hat dadurch acht flexible Segmente die beim Einführen und beim Herausnehmen, beziehungsweise beim Herausziehen des medizinischen Implantats, dieses abstreifen, das heißt die Oberfläche des Implantats überstreichen. Dadurch ist sichergestellt, dass der Abstreifer 6 im Wesentlichen die gesamte Oberfläche des medizinischen Implantats, insbesondere beim Herausziehen, überstreicht und dabei abstreift.

Im Inneren des Behälters 4 befindet sich eine Flüssigkeit 8, in die ein medizinisches Implantat eingetaucht werden kann. Über der Flüssigkeit 8 ist eine Membran 9 angeordnet, die den gesamten Querschnitt des Behälters 4 in seinem Inneren einnimmt. Die Flüssigkeit 8 ist eine wässrige Lösung mit Antibiotika, mit der ein medizinisches Implantat beschichtet werden soll. Die Membran 9 wird durch einen Halterungsring 10 getragen, der auf der Innenseite des Behälters 4 unterhalb des Abstreifers 6 angeordnet ist. Die Membran 9 schließt den Behälter 4 dicht ab, so dass keine Verunreinigung von außen in den Bereich unterhalb der Membran 9 vordringen kann.

Mit der gezeigten Vorrichtung 1 kann ein Verfahren durchgeführt werden. Ein medizinisches Implantat (nicht gezeigt) wird durch den Abstreifer 6 hindurch geschoben. Anschließend durchstößt das medizinische Implantat die Membran 9, die zuvor die darunter angeordnete Flüssigkeit 8 von äußeren Einflüssen bewahrt hat. Das medizinische Implantat wird dann in die Flüssigkeit 8 eingetaucht. Die Flüssigkeit 8 benetzt das medizinische Implantat. Anschließend wird das medizinische Implantat aus der Flüssigkeit 8 herausgezogen. Ein Teil der Flüssigkeit 8 bleibt am medizinischen Implantat haften.

Nachdem die Oberfläche des medizinischen Implantats beschichtet wurde, wird es aus dem Behälter 4 herausgezogen. Dabei wird die beschichtete Oberfläche des medizinischen Implantats am Abstreifer 6 vorbeigezogen. Überschüssige Flüssigkeit 8 wird dabei von der Oberfläche des medizinischen Implantats abgestreift. Das aus dem Behälter 4 herausgezogene medizinische Implantat ist dann zwar beschichtet, tropft aber nicht mehr nach. Durch diese Maßnahme kann verhindert werden, dass die Flüssigkeit 8 die Umgebung kontaminiert. Das mit der Flüssigkeit 8 beschichtete medizinische Implantat ist dann bereit, um für eine Operation eingesetzt zu werden.

Die Beschichtungsvorrichtung 1 ist aus Polypropylen gefertigt, hat eine Höhe von 25 cm und einen Durchmesser von 6 cm. Der Abstreifer 6 besteht ebenfalls aus Polypropylen. Die Membran 9 ist aus Aluminiumverbundfolie gebildet. Der Halterungsring 10 der Membran 9 ist aus Polypropylen gefertigt und ist im Presssitz in den Innenraum des Behälters 4 eingepresst. Der Behälter 4 kann vor der Anwendung zusätzlich durch eine Aluminiumverbundfolie (nicht gezeigt) keimdicht verschlossen sein, die die Öffnung des Behälters 4 verschließt.

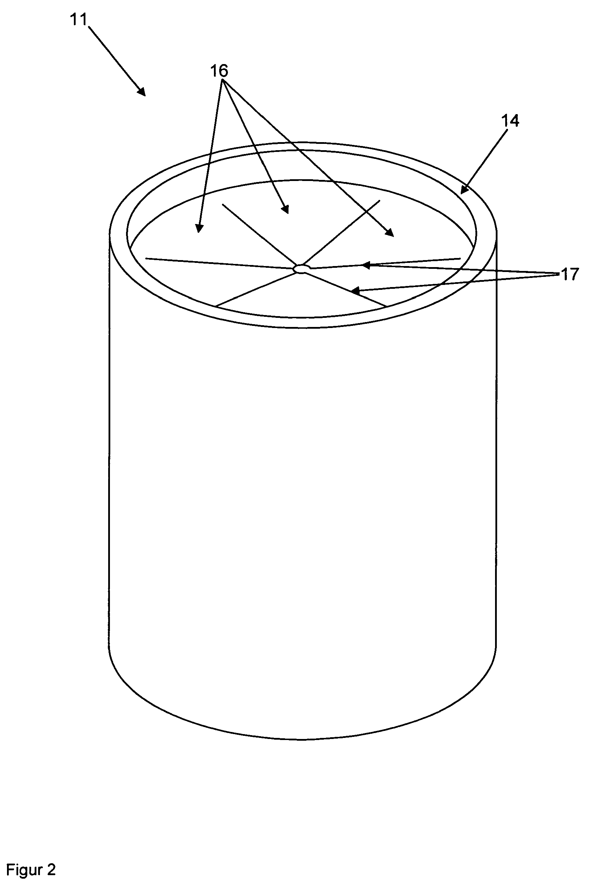

Figur 2 zeigt eine schematische perspektivische Ansicht einer zweiten erfindungsgemäßen Vorrichtung 11 für ein Verfahren. Die Vorrichtung 11 umfasst einen Behälter 14 und einen Abstreifer 16, der den Behälter 14 auf der Oberseite vollständig abdeckt. Der flexible Abstreifer 16 hat sechs Schlitze 17 beziehungsweise Einschnitte 17, die die Oberseite des Abstreifers 16 mit der ins Innere des Behälters 14 gewandten Unterseite des Abstreifers 16 verbinden, so dass ein medizinisches Implantat (nicht gezeigt) durch den Abstreifer 16 in das Innere des Behälters 14 entlang der dann umgeklappten Schlitze 17 eingeführt werden kann.

Im Inneren des Behälters 14 befindet sich eine Flüssigkeit (nicht gezeigt) in die ein medizinisches Implantat eingetaucht werden kann. Die Flüssigkeit enthält eine pharmazeutisch aktive Substanz, mit der das medizinische Implantat beschichtet ist, sobald das Lösungsmittel der Flüssigkeit, in der die pharmazeutisch aktive Substanz gelöst ist, verdampft ist.

Es können übliche Zweymüller-Hüftendoprothesen kurz in die mit Flüssigkeit befüllten Vorrichtungen 1, 21 bis zum Ende des Schafts hinein gesteckt und sofort wieder heraus gezogen werden. Die Zweymüller-Hüftprothesen haben dann einen Film einer Flüssigkeit 8 an der Schaftoberfläche. Nach dem Austrocknen des Flüssigkeitsfilms können die Zweymüller-Hüftprothesen dann einen weißen Belag an der Schaftoberfläche haben, in dem sich die pharmazeutisch aktive Substanz befindet. Die Hüftprothesen sind bereit zum Einsatz bei einer Operation.

Nach der Beschichtung mit der Flüssigkeit 8 kann das medizinische Implantat noch mit einem Pulver beschichtet werden, das in einem zweiten Behälter enthalten ist, in den das medizinische Implantat gesteckt werden kann. Das Pulver im zweiten Behälter ist vorzugsweise durch eine zweite Membran abgedeckt, die mit dem medizinischen Implantat durchstoßen und dadurch geöffnet wird. Das Pulver enthält eine knochenwachstumsfördernde Substanz, wie Calciumphosphat. Durch den Flüssigkeitsfilm auf dem medizinischen Implantat haftet das Pulver gut auf dessen Oberfläche. Daraus resultiert eine Flüssigkeits-Pulver-Beschichtung auf der zu beschichtenden Oberfläche des medizinischen Implantats.

Im Folgenden werden Beispiele zur Herstellung von Flüssigkeiten und von Pulvern für ein Verfahren und ein weiteres Beispiel für eine erfindungsgemäße Vorrichtung ausgeführt.

### Beispiel 1: Herstellung einer Beschichtungslösung mit Gentamicinsulfat:

Es wurden 16,0 g Gentamicinsulfat (Fujian Fukang Ltd.) mit 4,0 ml pyrogenfreiem sterilem Wasser bei Raumtemperatur vermischt. Nach 24 Stunden Rühren bei Raumtemperatur mit einem Magnetrührer hatte sich eine ölig-viskose gelbliche Lösung gebildet. Es ergab sich eine Beschichtungslösung mit Gentamicinsulfat als Flüssigkeit zur Beschichtung eines medizinischen Implantats.

### Beispiel 2: Herstellung einer Beschichtungslösung mit der Zweifachkombination Gentamicinsulfat und Clindamycinhydrochlorid:

Es wurden 12,0 g Gentamicinsulfat (Fujian Fukang Ltd.), 4,0 g Clindamycinhydrochlorid (Sigma-Aaldrich) mit 4,0 ml pyrogenfreiem sterilem Wasser bei Raumtemperatur vermischt. Nach 24 Stunden Rühren bei Raumtemperatur mit einem Magnetrührer hatte sich eine ölig-viskose gelbliche Lösung gebildet.

### Beispiel 3: Herstellung einer Beschichtungslösung mit der Dreifachkombination Gentamicinsulfat, Clindamycinhydrochlorid und Vancomycinhydrochlorid:

Es wurden 4,0 g Gentamicinsulfat (Fujian Fukang Ltd.), 4,0 g Clindamycinhydrochlorid (Sigma-Aaldrich) und 4,0 g Vancomycinhydrochlorid (Sigma-Aldrich) mit 8,0 ml pyrogenfreiem sterilem Wasser bei Raumtemperatur vermischt. Nach 24 Stunden Rühren bei Raumtemperatur mit einem Magnetrührer hatte sich eine viskose gelbliche Lösung gebildet.

### Beispiel 4: Herstellung einer Beschichtungslösung enthaltend Gentamicinsulfat und Äpfelsäure:

Es wurden 100 mg Äpfelsäure und 16,0 g Gentamicinsulfat (Fujian Fukang Ltd.) mit 4,0 ml pyrogenfreiem sterilem Wasser bei Raumtemperatur vermischt. Nach 24 Stunden Rühren bei Raumtemperatur mit einem Magnetrührer hatte sich eine ölig-viskose gelbliche Lösung gebildet.

### Beispiel 5: Herstellung einer Beschichtungslösung enthaltend Gentamicinsulfat und Citronensäure:

Es wurden 100 mg Citronensäure und 16,0 g Gentamicinsulfat (Fujian Fukang Ltd.) mit 4,0 ml pyrogenfreiem sterilem Wasser bei Raumtemperatur vermischt. Nach 24 Stunden Rühren bei Raumtemperatur mit einem Magnetrührer hatte sich eine ölig-viskose gelbliche Lösung gebildet.

### Beispiele 6 - 10: Beschichtung von medizinischen Implantaten:

Mit konventionellen 10 ml Kunststoffspritzen wurden jeweils 5 ml der Beschichtungslösungen der aufgeführten Beispiele 1 - 5 aufgezogen. Danach wurden mit den befüllten Kunststoffspritzen 4 ml der jeweiligen Wirkstofflösung in den Behälter einer erfindungsgemäßen Vorrichtung gespritzt. Medizinische Implantate wurden mit den in den Beispielen 1 - 5 beschriebenen Lösungen beschichtet, indem sie in den Behälter eingeführt und aus dem Behälter herausgenommen wurden. Es wurden jeweils medizinische Implantate erhalten, die mit den in den verwendeten Lösungen enthaltenen Antibiotika beschichtet waren.

### Beispiele 11 - 15: Beschichtung von medizinischen Implantaten mit Antibiotikum und knochenwachstumsstimulierenden Substanzen:

In den Beispielen 11 - 15 wurde gemäß den Beispielen 6 - 10 vorgegangen, wobei die medizinischen Implantate nach der Herausnahme aus dem Behälter in einen zweiten Behälter überführt und anschließend aus diesem herausgenommen wurden. Dieser, durch eine Membran verschlossene zweite Behälter war mit einem Pulvergemisch von 150 g Calciumsulfat-Hemihydrat (Siebfraktion < 64 µm), 15,0 g Calciumsulfat-Dihydrat (Siebfraktion < 64 µm) und 1,5 g Ammoniumsulfat (Siebfraktion < 64 µm) gefüllt.

*Beispiele 16* - *20: Beschichtung von medizinischen Implantaten mit Antibiotikum und anderen knochenwachstumsstimulierenden Substanzen:*In den Beispielen 16 - 20 wurde gemäß den Beispielen 6 - 10 vorgegangen, wobei die medizinischen Implantate nach der Herausnahme aus dem Behälter in einen zweiten Behälter überführt und anschließend aus diesem herausgenommen wurden. Dieser zweite Behälter war mit einem Pulvergemisch von 100 g Calciumsulfat-Hemihydrat (Siebfraktion < 64 µm), 50,0 g Calciumcarbonat (Siebfraktion < 64 µm), 15,0 g Calciumsulfat-Dihydrat (Siebfraktion < 64 µm) und 1,5 g Ammoniumsulfat gefüllt.

### Beispiele 21 - 25: Beschichtung von medizinischen Implantaten mit Antibiotikum und anderen knochenwachstumsstimulierenden Substanzen:

In den Beispielen 21 - 25 wurde gemäß den Beispielen 6 - 10 vorgegangen, wobei die medizinischen Implantate nach der Herausnahme aus dem Behälter in einen zweiten Behälter überführt und anschließend aus diesem herausgenommen wurden. Dieser zweite Behälter war mit einem 150 g ß-Tricalciumphosphat (Siebfraktion < 64 µm) gefüllt.

### Beispiele 26 - 30: Beschichtung von medizinischen Implantaten mit Antibiotikum und anderen knochenwachstumsstimulierenden Substanzen:

In den Beispielen 26 - 30 wurde gemäß den Beispielen 6 - 10 vorgegangen, wobei die medizinischen Implantate nach der Herausnahme aus dem Behälter in einen zweiten Behälter überführt und anschließend herausgenommen wurden. Dieser zweite Behälter war mit 150 g α-Tetracalciumphosphat (Siebfraktion < 64 µm) gefüllt.

In den Beispielen 11 - 30 wurden jeweils medizinische Implantate erhalten, die mit den verwendeten Antibiotika und den verwendeten knochenwachstumsfördernden Substanzen beschichtet waren.Die in der voranstehenden Beschreibung, sowie den Ansprüchen, Figuren und Ausführungsbeispielen offenbarten Merkmale der Erfindung können sowohl einzeln, als auch in jeder beliebigen Kombination für die Verwirklichung der Erfindung in ihren verschiedenen Ausführungsformen wesentlich sein.

### Bezugszeichenliste

- 1, 11: Vorrichtung
- 4, 14: Behälter
- 6, 16: Abstreifer
- 8: Flüssigkeit
- 9: Membran
- 10: Halterungsring
- 17: Schlitz / Einschnitt

## Patentansprüche

1. Vorrichtung (1, 11) zur zumindest bereichsweisen Beschichtung eines medizinischen Implantats **dadurch gekennzeichnet, dass** die Vorrichtung (1, 11) einen Behälter (4, 14) umfasst, der eine Flüssigkeit (8) aufweist, die zumindest eine pharmazeutisch aktive Substanz enthält, und der Behälter (4, 14) eine Öffnung zum Einführen und Herausnehmen des medizinischen Implantats umfasst, und dass über der Flüssigkeit (8) ein Übertragungsmittel angeordnet ist, mit dem eine zweite Flüssigkeit auf das medizinische Implantat übertragbar ist, wobei die zweite Flüssigkeit in dem Übertragungsmittel enthalten ist..

2. Vorrichtung (1, 11) nach Anspruch 1, **dadurch gekennzeichnet, dass** die Öffnung durch einen abziehbaren Deckel verschlossen ist.

3. Vorrichtung (1, 11) nach einem der Ansprüche 1 oder 2, **dadurch gekennzeichnet, dass** die Vorrichtung (1, 11) einen Abstreifer (6, 16) umfasst, der vorzugsweise im Bereich der Öffnung, insbesondere zwischen der Öffnung und der Flüssigkeit (8) angeordnet ist.

4. Vorrichtung (1, 11) nach Anspruch 3, **dadurch gekennzeichnet, dass** der Abstreifer (6, 16) scheibenförmig ist und mindestens einen Einschnitt (17) umfasst, der die Oberseite und die Unterseite der Scheibe (6) verbindet.

5. Vorrichtung (1, 11) nach Anspruch 4, **dadurch gekennzeichnet, dass** der Abstreifer (6, 16) als Kegelmantel (16) oder als Halbkugelfläche ausgebildet ist, wobei die Kegelspitze oder die Halbkugel in Richtung der Flüssigkeit (8) ausgerichtet ist und vorzugsweise der Kegelmantel (16) oder die Halbkugel mindestens einen Einschnitt (17) enthalten, der die Oberseite und die Unterseite des Abstreifers (6, 16) verbindet.

6. Vorrichtung (1, 11) nach einem der Ansprüche 1 bis 5, **dadurch gekennzeichnet, dass** die pharmazeutisch aktive Substanz Antibiotika und/oder organische Antiseptika in einer pharmazeutisch wirksamen Dosis beinhaltet.

7. Vorrichtung (1, 11) nach einem der Ansprüche 3 bis 6, **dadurch gekennzeichnet, dass** die Vorrichtung (1, 11) einen Vakuumanschluss umfasst, der mit einer Vakuumquelle verbindbar ist und der vorzugsweise zwischen dem Abstreifer (6, 16) und der Flüssigkeit (8) angeordnet ist.

8. Vorrichtung (1, 11) nach einem der Ansprüche 1 bis 7, **dadurch gekennzeichnet, dass** die Vorrichtung mit einem Pulver, mit einer Wirkstofflösung und/oder Wirkstoffsuspension vorbefüllt ist.

## Claims

1. Device (1, 11) for coating, at least regions of, a medical implant, **characterised in that**
the device (1, 11) comprises a container (4, 14) that comprises a liquid (8) that contains at least one pharmaceutically active substance, and the container (4, 14) comprises an opening for introducing and removing the medical implant, and **in that**
a transfer means is arranged above the liquid (8) that can be used to transfer a second liquid to the medical implant, whereby the second liquid is contained in the transfer means.

2. Device (1, 11) according to claim 1, **characterised in that** the opening is closed by a pull-off lid.

3. Device (1, 11) according to any one of the claims 1 to 2, **characterised in that** the device (1, 11) comprises a wiper (6, 16) that is preferably arranged in the region of the opening, in particular between the opening and the liquid (8).

4. Device (1, 11) according to claim 3, **characterised in that** the wiper (6, 16) is disc-shaped and comprises at least one notch (17) that connects the top and the bottom of the disc (6).

5. Device (1, 11) according to claim 4, **characterised in that** the wiper (6, 16) is shaped like an envelope of cone (16) or a hemispherical surface, whereby the tip of the cone or the hemisphere is oriented towards the liquid (8) and the envelope of cone (16) or the hemisphere preferably contain at least one notch (17) that connects the top and the bottom of the wiper (6, 16).

6. Device (1, 11) according to any one of the claims 1 to 5, **characterised in that** the pharmaceutically active substance contains antibiotics and/or organic antiseptic agents in a pharmaceutically effective dose.

7. Device (1, 11) according to any one of the claims 3 to 6, **characterised in that** the device (1, 11) comprises a vacuum connector that can be connected to a vacuum source and is preferably arranged between the wiper (6, 16) and the liquid (8).

8. Device (1, 11) according to any one of the claims 1 to 7, **characterised in that** the device is pre-filled with a powder, a solution of an active substance and/or a suspension of an active substance.

## Revendications

1. Dispositif (1, 11) pour revêtir, au moins par tronçons, un implant médical,
**caractérisé en ce que**
le dispositif (1, 11) comprend un récipient (4, 14) qui présente un liquide (8) qui contient au moins une substance pharmaceutique active, et le récipient (4, 14) comprend une ouverture pour introduire et prélever l'implant médical, et qu'un moyen de transfert est disposé au-dessus du liquide (8), avec lequel un second liquide peut être transféré sur l'implant médical, dans lequel le second liquide est contenu dans le moyen de transfert.

2. Dispositif (1, 11) selon la revendication 1, **caractérisé en ce que** l'ouverture est fermée par un couvercle amovible.

3. Dispositif (1, 11) selon la revendication 1 ou 2, **caractérisé en ce que** le dispositif (1, 11) présente une racle (6, 16) qui est disposée de préférence au niveau de l'ouverture, en particulier entre l'ouverture et le liquide (8).

4. Dispositif (1, 11) selon la revendication 3, **caractérisé en ce que** la racle (6, 16) est en forme de disque et comprend au moins une encoche (17) qui relie la face supérieure et la face inférieure du disque (6).

5. Dispositif (1, 11) selon la revendication 4, **caractérisé en ce que** la racle (6, 16) est conçue en tant qu'aire latérale de cône (16) ou en tant que surface hémisphérique, dans lequel la pointe du cône ou l'hémisphère est dirigée en direction du liquide (8) et de préférence, l'aire latérale de cône (16) ou l'hémisphère comprennent au moins une encoche (17) qui relie la face supérieure et la face inférieure de la racle (6, 16).

6. Dispositif (1, 11) selon l'une des revendications 1 à 5, **caractérisé en ce que** la substance pharmaceutique active contient des antibiotiques et/ou des antiseptiques organiques dans une dose active sur le plan pharmaceutique.

7. Dispositif (1, 11) selon l'une des revendications 3 à 6, **caractérisé en ce que** le dispositif (1, 11) comprend un raccordement à du vide qui peut être relié à une source de vide et qui est disposé de préférence entre la racle (6, 16) et le liquide (8).

8. Dispositif (1, 11) selon l'une des revendications 1 à 7, **caractérisé en ce que** le dispositif peut être pré-rempli avec une poudre, une solution de substance active et/ou une suspension de substance active.
